(19) Europäisches Patentamt
European Patent Office
Office européen des brevets

(11) EP 1 003 700 B1

(12) EUROPÄISCHE PATENTSCHRIFT

(45) Veröffentlichungstag und Bekanntmachung des Hinweises auf die Patenterteilung:
**19.06.2002 Patentblatt 2002/25**

(21) Anmeldenummer: **98947392.1**

(22) Anmeldetag: **07.08.1998**

(51) Int Cl.[7]: **C07C 29/34**, C07C 29/80

(86) Internationale Anmeldenummer:
**PCT/DE98/02345**

(87) Internationale Veröffentlichungsnummer:
**WO 99/07661 (18.02.1999 Gazette 1999/07)**

(54) **VERFAHREN ZUR HERSTELLUNG VON METALLFREIEN GUERBETALKOHOLEN**
METHOD FOR PRODUCING METAL-FREE GUERBET ALCOHOLS
PROCEDE DE FABRICATION D'ALCOOLS DE GUERBET EXEMPTS DE METAUX

(84) Benannte Vertragsstaaten:
**AT BE DE ES FR GB IT NL**

(30) Priorität: **11.08.1997 DE 19734673**

(43) Veröffentlichungstag der Anmeldung:
**31.05.2000 Patentblatt 2000/22**

(73) Patentinhaber: **SASOL Germany GmbH**
**22297 Hamburg (DE)**

(72) Erfinder:
- **SCHERF, Erich**
**D-25541 Brunsbüttel (DE)**
- **LETSCH, Hans-Jürgen**
**D-25541 Brunsbüttel (DE)**
- **SCHRÖDER, Clemens**
**D-23863 Kayhude (DE)**
- **HERRMANN, Albert, Thomas**
**D-25541 Brunsbüttel (DE)**

(74) Vertreter: **Schupfner, Georg U. et al**
**Müller, Schupfner & Gauger,**
**Patentanwälte,**
**Postfach 1753**
**21236 Buchholz (DE)**

(56) Entgegenhaltungen:
**DE-A- 4 014 736**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 003 700 B1

Printed by Jouve, 75001 PARIS (FR)

## Beschreibung

Die Erfindung betrifft ein Verfahren zur Herstellung von Guerbetalkohole hoher Reinheit durch Kondensation primärer und / oder sekundärer Alkohole mit 2 bis 30 Kohlenstoffatomen in Gegenwart eines oder mehrerer alkalischer Katalysatoren und / oder eines oder mehrerer Schwermetall-Katalysatoren.

Guerbetalkohole sind bekannte Verbindungen, die als Grundstoffe für viele Anwendungen wie z.B. in der kosmetischen-, der pharmazeutischen-, der Textil- oder der Schmiermittelindustrie eingesetzt werden.

Guerbetalkohole lassen sich durch Kondensation primärer und sekundärer Alkohole in Gegenwart von starken Alkalibasen nach folgendem Reaktionsschema herstellen:

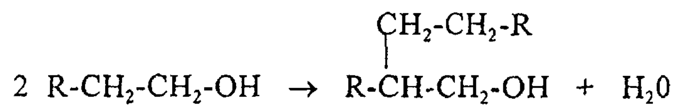

Eine Vielzahl von Katalysator-Systemen auf Basis von Alkali-/Erdalkalisalzen in Gegenwart von Schwermetallen als Cokatalysatoren ist bekannt.

Als alkalische Katalysatoren sind Alkalimetalle, Alkalimetallhydroxide, Alkalimetalloxide oder Alkalimetallalkoholate bekannt. Weiterhin sind Kombinationen von z B. KOH mit ZnO beschrieben worden. Diese weisen jedoch oft spezifische Nachteile auf (siehe z.B. Soap/Cosmetics/Chemical Specialties, Seiten 52-55 und 115, April 1987).

Als Schwermetall-Cokatalysatoren sind ZnO, PbO, NiO, Pd sowie Ti- und Zr-Verbindungen beschrieben worden. Die Deutsche Patentschrift DE 24 00 326 beschreibt eine Vielzahl möglicher Cokatalysatoren. Gemäß der Deutschen Patentschrift DE 26 34 676 werden als mögliche Katalysatoren weiterhin unlösliche Salze aus der Gruppe der Bleisilikate, Bleititanate und Bleizirkonate in Gegenwart von Alkalibasen genannt.

Ebenso sind Cokatalysatoren auf Basis von ZnO und unlöslichen Bleisalzen aus der Gruppe der Bleisilikate, Bleititanate und Bleizirkonate bekannt. Die Bleisalze können beliebige Verhältnisse von PbO zu $SiO_2$, $TiO_2$ bzw. $ZrO_2$ aufweisen, was gleichzeitig eine unterschiedliche Basizität der Cokatalysatoren bedingt.

Weiterhin sind heterogene Katalysatorsysteme bekannt wie z.B. Katalysatorsysteme bestehend aus Platin auf einem aktivierten Kohlenstoffträger mit hoher Oberfläche und Kalium- oder Natriumhydroxid.

Auch wenn durch den Einsatz geeigneter Katalysatorsysteme die Umsetzungsgeschwindigkeit und die Ausbeute der eingesetzten Alkohole zu den Guerbetalkoholen erheblich erhöht werden kann, stellen die eingesetzten Schwermetallsalze erhöhte Anforderungen an deren Abtrennung wegen der damit verbundenen Umweltprobleme bezüglich ihrer Entsorgung dar.

Eine weitere Schwierigkeit besteht darin, daß das Rohprodukt nach der Guerbetreaktion üblicherweise Nebenprodukte, wie Aldehyde, ungesättigte Verbindungen und insbesondere Seifen in variierenden Mengen enthält. Dabei ist es besonders wichtig, daß die im Reaktionsgemisch bei Raumtemperatur löslichen und unlöslichen Seifen nach beendeter Reaktion abgetrennt werden. Die Abtrennung der Seifen erfolgt in der Regel durch Auswaschen mit teilweise angesäuerten wäßrigen Lösungen, z. B. 6%iger Natriumchloridlösung (DE-A1-26 34 676). Bei diesem Verfahren fallen große Mengen Abwasser an, die gleichzeitig die Schwermetalle enthalten. Eine nachträgliche Ausfällung und Abtrennung der als Katalysatoren bzw. Cokatalysatoren eingesetzten Schwermetalle ist mit einem erheblichen Aufwand verbunden. Das Auswaschen mit wäßrigen Lösungen hat außerdem den Nachteil, daß Abwasser mit hoher organischer Fracht anfällt, was zudem zu Ausbeuteverlusten der Guerbetalkohole führt. Es wäre zwar denkbar, die Cokatalysatoren auf feste Träger, wie z B. Aktivkohle, aufzubringen, die durch Filtration nach der Reaktion abgetrennt werden können. Dem steht jedoch die Feststellung entgegen, daß die Träger sich während der Reaktion mechanisch und auch chemisch zersetzen, so daß eine vollständige Abtrennung durch einfaches Abfiltrieren nicht möglich ist. Desweiteren führt die Herstellung von Cokatalysatoren auf Trägern im Gegensatz zur üblichen direkten Verwendung von Übergangsmetallsalzen und -oxiden zu einer deutlichen Erhöhung der Kosten.

In der DE-A-40 14 736 wird eine Verfahren mit Kaskadenreaktor und Kondensationaufsatz unter Eduktrückführung und Kondensationswasserabscheidung beschreiben. Das erhaltene Kondensationsprodukt ist metall- und seifenhaltig und muß nach Ausbringen aus dem Kaskadenreaktor mit Hilfe herkömmlicher Maßnahmen gereinigt werden.

In der DE 195 31 714 wird zum Vermeiden dieser Probleme vorgeschlagen, die bei Raumtemperatur schlecht löslichen Seifen durch Filtration, Zentrifugation und / oder Extraktion zu entfernen und anschließend zu Destillieren. Dabei fällt jedoch bei der Filtration ein Filterkuchen an, welcher zu erhöhten Abfallmengen führt. Bei der beschriebenen Extraktion mit Wasser fällt wieder ein stark schwermetall-belastetes Abwasser an. bei der Filtration ein Filterkuchen an, welcher zu erhöhten Abfallmengen führt. Bei der beschriebenen Extraktion mit Wasser fällt wieder ein stark schwermetall-belastetes Abwasser an.

Der vorliegenden Erfindung lag die Aufgabe zugrunde, ein wirtschaftliches Verfahren zur Herstellung von Guerbetalkoholen zur Verfügung zu stellen, welche Abwässer, die nach herkömmlicher Aufarbeitung anfallen, vollständig vermeidet und metall- und seifenfreie Produkte liefert.

Gegenstand der Erfindung ist ein Verfahren zur Herstellung von Guerbetalkoholen hoher Reinheit, inbesondere seifen- und schwermetallfreien Guerbetalkoholen, durch Kondensation primärer und / oder sekundärer Alkohole mit 2 bis 30 Kohlenstoffatomen in Gegenwart eines oder mehrerer alkalischer Katalysatoren und/oder eines oder mehrerer Schwermetall-Katalysatoren, indem man das Reaktionsprodukt unmittelbar, d.h. ohne dazwischen geschaltete andersartige Reinigungsschritte, durch Destillation in Produkt-Alkohol sowie ggf. vorhandenen Edukt-Alkohol und auf der anderen Seite Katalysator / Katalysator-Gemisch sowie ggf. vorhandene höhermolekulare Produkte trennt, wobei das Reaktionsprodukt sowohl batchweise wie auch kontinuierlich dem Reaktionsraum entnommen werden kann.

Das Reaktionsprodukt wird ohne Auswaschen, Filtrieren, Zentrifugieren, Wasserdampfdestillation oder andere Reinigungsschritte direkt einer Destillation zugeführt, wobei sich ein bei Raumtemperatur hochviskoser Sumpf bildet. Dieser Sumpf läßt sich einfach entsorgen.

Überraschenderweise wurde festgestellt, daß bei dem erfindungsgemäßen Verfahren das Reaktionsgemisch aus der Guerbetreaktion durch eine einfache und damit auch wirtschaftliche Weise von den Schwermetallionen und alkalischen Katalysatorresten befreit werden kann, indem man das rohe Reaktionsprodukt destilliert. Durch diesen Verfahrensschritt wird das Auftreten von Abwasser und somit eine Abwasserbelastung mit Schwermetallionen vermieden. Als Cokatalysatoren werden handelsübliche Schwermetallsalze eingesetzt. Es kann auf den Einsatz von auf Trägermaterialien befindlichen Verbindungen verzichtet werden. Desweiteren werden schwermetallfreie Produkte erhalten, was z.B. für den Einsatz in Pharmazie und Kosmetik von extremer Wichtigkeit ist.

Erfindungsgemäß werden primäre und / oder sekundäre, lineare oder cyclische Alkanole mit 2 bis 30 Kohlenstoffatomen und solche mit einer Methylengruppe in $\alpha$-Stellung zu dem die Hydroxylgruppe tragenden Kohlenstoffatom zu den gewünschten, im wesentlichen von höhermolekularen Kondensationsprodukten freien Guerbetalkoholen umgesetzt. Derartige Edukt-Alkanole können durch die allgemeine Formel

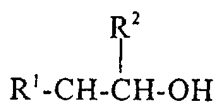

wiedergegeben werden. Die Reste $R^1$ und $R^2$ können für ein Wasserstoffatom, einen Arylrest oder eine geradkettige oder verzweigte Alkylgruppe stehen. Dabei können $R^1$ und $R^2$ gleich oder verschieden sein. Vorteilhaft sind solche Ausgangsverbindungen, in denen $R^1$ für eine Alkylgruppe und $R^2$ für ein Wasserstoffatom steht, d.h. primäre Alkan-1-ole. Typische Beispiele für derartige Alkanole (jeweils mit endständiger OH-Funktion) sind Ethanol, Propanol, Isopropanol, Butanol, Pentanol, Hexanol, Octanol, Decanol, Undecanol, Dodecanol, Tridecanol, Tetradecanol, Pentadecanol, Hexadecanol, Octadecanol, Eicosanol, Docosanol, Tetracosanol, Hexacosanol, Octacosanol und Triacontanol; weiterhin sekundäre Alkanole wie 4-Methylpentan-2-ol, Hexan-2-ol, Octan-2-ol, Cyclopentanol, Cyclohexanol sowie die entsprechenden Isomere der oben genannten primären Alkanole.

Die vorgenannten Ausgangsverbindungen können synthetischen Ursprungs sein, z.B. sogenannte Ziegler- oder Oxoalkohole. Sie können jedoch auch natürlicher Herkunft sein. Besonders bevorzugte Ausgangsverbindungen sind geradkettige primäre Alkanole mit 6 bis 22 Kohlenstoffatomen; typische Beispiele hierfür sind Capron-, Önanth-, Capryl-, Pelargon-, Caprin-, Lauryl-, Myristyl-, Cetyl-, Stearyl-, Arachidyl- und Behenylalkohol.

Die vorgenannten Ausgangsmaterialien können selbstverständlich auch, wie in der Fettchemie üblich, in Form ihrer technischen Gemische mit anderen Alkoholen eingesetzt werden.

Als Katalysatoren, die zur Durchführung des erfindungsgemäßen Verfahrens eingesetzt werden können, eignen sich die aus dem Stand der Technik bekannten Katalysatoren. Als alkalische Katalysatoren eignen sich besonders die Oxide, Hydroxide und Alkoholate der Alkalimetalle Lithium, Natrium, Kalium und Cäsium.

Besonders bevorzugt werden Kaliumhydroxid und / oder Cäsiumhydroxid eingesetzt.

Als Cokatalysatoren, die zur Durchführung des erfindungsgemäßen Verfahrens eingesetzt werden können, eignen sich die aus dem Stand der Technik bekannten Cokatalysatoren, wie sie z.B. in der DE-A1-24 00 326 aufgezählt werden.

Die Cokatalysatoren können in einer Menge von 0,05 g bis 3,0 g pro Mol, bezogen auf die Gesamtmenge des eingesetzten Alkohols, eingesetzt werden.

Erfindungsgemäß erfolgt die Abtrennung der Seife und Schwermetalle von den Roh-Guerbetalkoholen ohne das nach dem Stand der Technik übliche Auswaschen oder eine Wasserdampf-Destillation (US 2,457,866). Unter Auswaschen ist dabei die Behandlung der Roh-Guerbetalkohole mit einem Überschuß an wäßrigen Lösungen zur

Entfernung der Nebenprodukte und Seifen zu verstehen, wobei große Mengen an metallhaltigem, mit organischen Lösungsmitteln beladenem Abwasser anfallen. Desweiteren ist es auch nicht erforderlich, eine Filtration, Extraktion oder Zentrifugation durchzuführen.

Die optimale Temperatur zur Herstellung der Guerbetalkohole gemäß dem erfindungsgemäßen Verfahren hängt von der Art des Ausgangsalkohols ab. Im allgemeinen kann die Reaktion mit gutem Erfolg bei der Siedetemperatur des Alkohols durchgeführt werden. Wenn auch Temperaturen von etwa ab 180°C angewendet werden können und damit zufriedenstellende Resultate erhalten werden, so wird hier eine Temperatur von 200 bis 320 °C bevorzugt und insbesondere eine Temperatur von 250°C bis 320°C, gelegentlich auch bis 350°C, gewählt, um das Reaktionswasser so schnell, wie es sich bildet, aus dem Gleichgewicht der Reaktion zu entfernen. Hierdurch werden hohe Umsätze und Ausbeuten erzielt. Die obere Temperaturgrenze ist die Temperatur, bei welcher der Ausgangsalkohol oder das Reaktionsprodukt sich zu zersetzen beginnen. Im allgemeinen liegt diese Grenze bei etwa 350 bis 400° C. Normalerweise ist eine Reaktionstemperatur von 250 bis 320°C in der Reaktionsmischung für gute Resultate ausreichend.

Im allgemeinen wird die Umsetzung bei Atmosphärendruck ausgeführt. Wenn allerdings der Siedepunkt des Ausgangsalkohols unterhalb der optimalen Reaktionstemperatur liegt, kann die Reaktion auch in einem abgeschlossenen System bei Drücken bis zu 30 bar durchgeführt werden. Bevorzugt ist der Druck hoch genug, um das Reaktionssystem in flüssiger Phase zu halten. Wenn die Reaktion in einem abgeschlossenen System bei erhöhtem Druck durchgeführt wird, muß dafür gesorgt werden, daß alles Reaktionswasser, das während des Ablaufs der Reaktion gebildet wird, sofort entfernt wird, um hohe Ausbeuten zu gewährleisten. Werden langkettige Alkohole, wie z.B. Tetradecanol, eingesetzt, so ist es erforderlich, mit Unterdruck das Reaktionswasser zu entfernen. Die für die Reaktion erforderliche Zeit ist nicht sonderlich kritisch. Sie kann in weiten Grenzen variiert werden, wobei lediglich darauf geachtet werden muß, daß die Reaktionszeit ausreichend lang bemessen wird, um die Bildung von dimeren Produkten zu gewährleisten, aber nicht so lange dauern sollte, daß weitere Kondensationen zu trimeren Produkten stattfinden. Im allgemeinen ist diese eine mittlere Verweilzeit von 0,5 Stunden bis 4 Stunden, bevorzugt wählt man zwischen 1,5 und 3 Stunden Reaktionszeit bei gegebener Temperatur, Druck und Katalysatorkonzentration.

Das Reaktionsgemisch wird zur Reinigung destilliert. Im Gegensatz zu den aus dem Stand der Technik bekannten Verfahren bleibt der Destillationsrückstand so flüssig, daß er noch förderbar ist. Ein weiterer Vorteil ist, daß die Destillationsblase sich nicht mit Feststoffen zusetzt, so daß entsprechende Reinigungsprobleme entfallen. Insgesamt wird die Ausbeute an Wertprodukten, Vorlaufalkohol, der recyclierbar ist, und Guerbetalkohol über alle Verfahrensstufen erhöht. Der Destillationsrückstand kann leicht entsorgt werden.

Die Destillation wird in zumindest zwei Stufen durchgeführt, worin in der ersten Stufe durch eine durch Entspannung herbeigeführte Flashdestillation im wesentlichen vom Eduktalkohol abgetrennt wird, wobei man vorzugsweise im wesentlichen ohne Zufuhr von Wärmeenergie bei im wesentlichen unveränderter Temperatur den Reaktoraustrag destilliert. In der zweiten Stufe werden, vorzugsweise durch Dünnschicht- bzw. Kurzwegdestillation, im wesentlichen Produktalkohol und Katalysator voneinander getrennt.

Die mittlere Verweilzeit des Reaktionsproduktes bis zur Abtrennung des Katalysators / Katalysator-Gemisches in der (den) Destillationvorrichtung(en) beträgt vorzugsweise 0,1 bis 10 min, besonders bevorzugt unter 6 min. Weiterhin destilliert man bevorzugt in zumindest einer, vorzugsweise beiden, Destillationsstufen bei einem Vakuum von 1 - 100 mbar, besonders bevorzugt 5 bis 50 mbar, und Temperaturen von 200 - 320 °C. Man destilliert vorzugsweise so, daß der verbleibende Katalysatorsumpf bei Temperaturen größer 200 °C flüssig förderbar ist.

Zur Vermeidung von Nebenproduktbildungen wird die Reaktion abgebrochen, indem die organischen Verbindungen aus dem Rohprodukt so schnell wie technisch realisierbar destillativ entfernt werden.

Das Rohprodukt wird hierzu - aus dem Reaktor kommend - vorzugsweise zunächst durch eine Flash-Entspannung in eine Monomeralkohol (Edukt) und Dimeralkoholfraktion (Produkt) getrennt. Bei der Flash-Destillation werden flüssige und dampfförmige Phase miteinander ins Gleichgewicht gesetzt und sodann die Gasphase abgezogen. Die dampfförmige Monomerfraktion (der in 2- Position unsubstituierter Eduktalkohol) wird kondensiert, leicht unterkühlt und direkt in den Reaktor zurückgeführt. Die alkalische Dimerfraktion bleibt bei der Entspannung flüssig und wird zur Abtrennung der alkalischen Nebenprodukte in einem Dünnschichtverdampfer destilliert. Als Dünnschichtdestillation im Sinne dieser Erfindung werden auch Film-, Fallfilm- bzw. Kurzwegdestillation angesehen. Bei der Dünnschichtverdampfung wird die zu verdampfende Flüssigkeit in dünnen Schichten von unter 0,3 mm, vorzugsweise unter 0,2 mm, Dicke durch mechanische Einwirkung (z.B. Gravitationskraft, Zentrifugalkraft, mechanische Wischer) verteilt und bei reduziertem Druck zur Verdampfung gebracht.

Die Flash-Destillation ist gekennzeichnet durch eine bevorzugte Verweilzeit von 0,1 bis 5 min und eine bevorzugte Destillationstemperatur von 200 bis 320°C bei 5 bis 100 mbar. Die Dünnschicht- bzw. Kurzwegdestillation ist gekennzeichnet durch eine bevorzugte Verweilzeit von 0,1 bis 5 min und eine bevorzugte Destillationstemperatur von 200 bis 320°C bei 5 bis 100 mbar.

Die alkalischen Nebenprodukte sind weitestgehend frei von Dimeralkohol und werden nach Austritt aus dem Dünnschichtverdampfer auf Umgebungstemperatur gekühlt und in hochviskoser Konsistenz entsorgt.

Die nicht mehr alkalische Dimerfraktion wird nach Verlassen des Dünnschichtverdampfers kondensiert und ggf. weiter destillativ , z.B. durch eine herkömmliche fraktioniert Destillation, gereinigt. Hierbei fallen eine leichte Zwischenfraktion sowie höhere organische Verbindungen als Nebenprodukte und Monomeralkohol, der in den Reaktionsprozeß zurückgeführt wird, an.

**Beispiele**

1. Herstellung von 2-Butyloctanol aus 1-Hexanol

Unter den in der Verfahrensbeschreibung aufgezeigten Bedingungen wurde ein Rohguerbetalkoholprodukt erhalten. Dieses wurde ungekühlt kontinuierlich in einen Flash-Behälter auf 50 mbar entspannt. Es stellte sich im Flash-Behälter die Gleichgewichtstemperatur ein. Dabei wurde der größte Teil des Hexan-1-ols als Kopfprodukt abgetrennt und der Reaktion wieder zugeführt. Die Verweilzeit wurde im Flash-Behälter auf kleiner 5 min. eingestellt.

Die alkalische katalysatorhaltige Dimerfraktion wurde direkt in einen Dünnschichtverdampfer überführt. Bei 30 mbar und Temperaturen im Bereich von 250 bis 290 °C wurde das Reaktionsprodukt vom Katalysatorsystem, höheren Oligomeren und Metallseifen befreit. Das hierbei anfallende Sumpfprodukt war frei von eingesetztem 1-Hexanol und gebildetem 2-Butyloctanol. Das Sumpfprodukt war unter den Destillationsbedingungen flüssig förderbar. Dieses wurde ausgetragen, abgekühlt und in hochviskoser Konsistenz der Entsorgung zugeführt. 2-Butyloctanol wurde metall- und alkalifrei als Wertprodukt in hoher Ausbeute erhalten.

2. Herstellung von 2-Octyldodecanol aus 1-Decanol

Unter den in der Verfahrensbeschreibung aufgezeigten Bedingungen wurde ein Rohguerbetalkoholprodukt erhalten. Dieses wurde ohne Kühlung kontinuierlich in einen Flash-Behälter auf 50 mbar entspannt. Es stellte sich im Flash-Behälter die Gleichgewichtstemperatur ein. Dabei wird der größte Teil des 1 Decanol als Kopfprodukt abgetrennt und der Reaktion wieder zugeführt. Die Verweilzeit im Flash-Behälter wurde auf kleiner 5 min. eingestellt.

Die alkalische katalysatorhaltige Dimerfraktion wurde direkt in einen Dünnschichtverdampfer überführt. Bei 5 mbar und Temperaturen im Bereich von 250 bis 290 °C wurde das Reaktionsprodukt vom Katalysatorsystem, höheren Oligomeren und Metallseifen befreit. Das hierbei anfallende Sumpfprodukt war frei von eingesetztem 1-Decanol und gebildetem 2-Octyldodecanol. Das Sumpfprodukt war unter den Destillationsbedingungen flüssig förderbar. Dieses wurde ausgetragen, abgekühlt und in hochviskoser Konsistenz der Entsorgung zugeführt. 2-Octyldodecanol wurde metall- und alkalifrei als Wertprodukt in hoher Ausbeute erhalten.

**Patentansprüche**

**1.** Verfahren zur Herstellung von Guerbetalkoholen hoher Reinheit durch Kondensation primärer und / oder sekundärer Alkohole mit 2 bis 30 Kohlenstoffatomen in Gegenwart eines oder mehrerer alkalischer Katalysatoren und / oder eines oder mehrerer Schwermetall-Katalysatoren, **dadurch gekennzeichnet, daß** man das Reaktionsprodukt nach Entnahme aus dem Reaktionsraum unmittelbar durch eine zumindest zweistufige Destillation

- in Produkt-Alkohol mit ggf. noch vorhandenem Edukt-Alkohol und
- Katalysator / Katalysator-Gemisch mit ggf. vorhandenen höhermolekularen Produkten trennt,

wobei die erste Destillationsstufe eine durch Entspannung herbeigeführte Flashdestillation ist.

**2.** Verfahren nach Anspruch 1, **dadurch gekennzeichnet, daß** die mittlere Verweilzeit des Reaktionsproduktes bis zur Abtrennung des Katalysators / Katalysator-Gemisches in der (den) Destillationvorrichtung(en) 0,1 bis 10 min bevorzugt 0,1 bis 6 min beträgt.

**3.** Verfahren nach einem der vorhergehenden Ansprüche, **dadurch gekennzeichnet, daß** man in der ersten Destillationsstufe im wesentlichen den Eduktalkohol vom Reaktionsgemisch Produktalkohol / Katalysator und in der zweiten Stufe im wesentlichen den Produktalkohol vom Katalysator abtrennt.

**4.** Verfahren nach einem der vorhergehenden Ansprüche, **dadurch gekennzeichnet, daß** die zweite Destillation eine Dünnschicht- bzw. Kurzwegdestillation ist.

**5.** Verfahren nach einem der vorhergehenden Ansprüche, **dadurch gekennzeichnet, daß** man zumindest in einer,

vorzugsweise in beiden, Destillationsstufen bei einem Vakuum von 1 - 100 mbar, vorzugsweise 5 bis 50 mbar, und Temperaturen von 200 - 320 °C destilliert.

6. Verfahren nach einem der vorhergehenden Ansprüche, **dadurch gekennzeichnet; daß** man so destilliert, daß der Katalysatorsumpf Temperaturen größer 200 °C aufweist und flüssig förderbar ist.

7. Verfahren nach einem der vorhergehenden Ansprüche, **dadurch gekennzeichnet, daß** man in einer dritten Destillationsstufe einen eduktarmen katalysatorfreien Produktalkohol fraktioniert destilliert, vorzugsweise kontinuierlich fraktioniert destilliert.

8. Verfahren nach einem der vorhergehenden Ansprüche, **dadurch gekennzeichnet, daß** das zu reinigende Reaktionsprodukt hergestellt ist, indem man die Reaktion zur Herstellung der Guerbetalkohole bei Temperaturen im Bereich von 200 bis 320°C, besonders bevorzugt bei 250 bis 320 °C, und Drücken im Bereich von 50 mbar bis 30 bar durchführt.

9. Verfahren nach einem der vorhergehenden Ansprüche, **dadurch gekennzeichnet, daß** das zu reinigende Reaktionsprodukt hergestellt ist, indem man geradkettige primäre oder cyclische Alkanole mit 6 bis 22 Kohlenstoffatomen kondensiert.

**Claims**

1. Process for producing high-purity Guerbet alcohols by condensation of primary and/or secondary alcohols having 2 to 30 carbon atoms in the presence of one or more alkaline catalyst(s) and/or one or more heavy metal catalyst(s), **characterised in that** the reaction product after removal from the reaction space is immediately split up by a two stage distillation

   - into product alcohol, optionally containing some educt alcohol, and
   - catalyst/ catalyst mixture, optionally containing some higher-molecular products,
   - the distillation in the first stage being a flash distillation induced by expansion.

2. Process according to claim 1,
**characterised in that** the mean residence time of the reaction product until the catalyst/catalyst mixture is separated in the distillation apparatus(es) is 0.1 to 10 minutes, preferably 0.1 to 6 minutes.

3. Process according to any one of the preceding claims,
**characterised in that** in the first distillation stage primarily educt alcohol is separated from the product alcohol/ catalyst reaction mixture, while in the second stage mainly product alcohol is separated from the catalyst.

4. Process according to any one of the preceding claims,
**characterised in that** the second distillation is a thin-layer or molecular distillation.

5. Process according to any one of the preceding claims,
**characterised in that** in at least one of the distillation stages, preferably in both the distillation is conducted at a vacuum of 1 to 100 mbar, preferably 5 to 50 mbar, and temperatures of 200 to 320 °C.

6. Process according to any one of the preceding claims,
**characterised in that** the distillation is conducted such that the catalyst bottoms have temperatures of higher than 200 °C and are pumpable as a liquid.

7. Process according to any one of the preceding claims,
**characterised in that** in a third distillation stage a catalyst-free product alcohol containing little educt is subjected to fractional distillation which is preferably conducted continuously.

8. Process according to any one of the preceding claims,
**characterised in that** the reaction product to be purified is obtained by carrying out the reaction for producing Guerbet alcohols at temperatures of 200 °C to 320 °C, most preferably 250 °C to 320 °C, and pressures of 50 mbar to 30 bar.

**9.** Process according to any one of the preceding claims,
**characterised in that** the reaction product to be purified is produced by condensing straight-chain, primary or cyclic alkanols having 6 to 22 carbon atoms.

## Revendications

**1.** Procédé pour la préparation d'alcools de Guerbet de pureté élevée par condensation d'alcools primaires et/ou secondaires comprenant 2 à 30 atomes de carbone en présence d'un ou de plusieurs catalyseurs de métal alcalin et/ou d'un ou de plusieurs catalyseurs de métal lourd, **caractérisé en ce qu'**on sépare le produit de réaction après soutirage de l'espace de réaction, directement par une distillation au moins en deux étapes, en

- alcool produit, avec de l'alcool de départ le cas échéant encore présent, et
- catalyseur/mélange de catalyseurs, avec des produits de haut poids moléculaire le cas échéant présents,

la première étape de distillation étant une distillation flash provoquée par une détente.

**2.** Procédé selon la revendication 1, **caractérisé en ce que** le temps de séjour moyen du produit de réaction jusqu'à la séparation du catalyseur/mélange de catalyseurs dans l'installation ou les installations de distillation est de 0,1 à 10 minutes, de préférence de 0,1 à 6 minutes.

**3.** Procédé selon l'une quelconque des revendications précédentes, **caractérisé en ce qu'**on sépare dans la première étape de distillation essentiellement l'alcool de départ du mélange réactionnel alcool produit/catalyseur et dans la deuxième étape essentiellement l'alcool produit du catalyseur.

**4.** Procédé selon l'une quelconque des revendications précédentes, **caractérisé en ce que** la deuxième distillation est une distillation en couche mince ou, selon le cas, une distillation directe.

**5.** Procédé selon l'une quelconque des revendications précédentes, **caractérisé en ce qu'**au moins dans une étape, de préférence dans les deux étapes de distillation, on distille sous un vide de 1 à 100 mbars, de préférence de 5 à 50 mbars et à des températures de 200 à 320°C.

**6.** Procédé selon l'une quelconque des revendications précédentes, **caractérisé en ce qu'**on distille de telle manière que le résidu de distillation de catalyseur présente des températures supérieures à 200°C et puisse être transporté sous forme liquide.

**7.** Procédé selon l'une quelconque des revendications précédentes, **caractérisé en ce qu'**on distille par fractionnement, de préférence par fractionnement continu, dans une troisième étape de distillation un alcool produit pauvre en produit de départ; exempt de catalyseur.

**8.** Procédé selon l'une quelconque des revendications précédentes, **caractérisé en ce que** le produit de réaction à purifier est préparé par le fait qu'on effectue la réaction pour la préparation des alcools de Guerbet à des températures dans la plage de 200 à 320°C, de manière particulièrement préférée de 250 à 320°C et à des pressions dans la plage de 50 mbars à 30 bars.

**9.** Procédé selon l'une quelconque des revendications précédentes, **caractérisé en ce que** le produit de réaction à purifier est préparé par le fait qu'on condense des alcanols linéaires primaires ou cycliques, comprenant 6 à 22 atomes de carbone.